# EUROPEAN PATENT SPECIFICATION

(11) **EP 0 591 240 B1**
(45) Date of publication and mention of the grant of the patent: **10.04.1996**
(21) Application number: 92910545.0
(22) Date of filing: 18.05.1992
(51) Int. Cl.: G01N 27/12, G01N 33/00

(54) **A GAS SENSOR**
Gassensor
DETECTEUR DE GAZ

(30) Priority: 18.05.1991 GB 9110797
(43) Date of publication of application: 13.04.1994
(73) Proprietor: CAPTEUR SENSORS & ANALYSERS LTD., Abingdon OX14 4RY (GB)
(72) Inventor: WILLIAMS, David, Edward, Abingdon OX14 2EE (GB)
(74) Representative: Wendon, James
(86) International application number: GB9200897
(87) International publication number: WO9221018

(56) References cited:
- WO-A-90/03569
- US-A- 4 453 397

## Description

This invention relates to methods for detecting at least one gaseous medium in the presence of at least one other gaseous medium, and to gas sensors suitable for use in such methods.

It is well known that the electrical conductivities of metal oxide semiconducting materials are sensitive to the presence of various gases or vapours and can be used in sensors to detect their presence. See for example the documents GB-A-2 149 120; GB-A-2 149 121; GB-A-2 149 122; GB-A-2 149 123; GB-A-2 166 244 and GB-A-2 218 523, and papers "The Tin Oxide Gas Sensor and its applications", J. Watson, Sensors and Actuators 54(1984) 29-42; "The Detection and Measurement of CO using ZnO Single Crystals", B. Bott et al, Sensors and Actuators 5(1984) 65-73; "The Role of Catalysis in Solid State Gas Sensors", S. J. Gentry et al, Sensors and Actuators 10(1986) 141-163; "Selectivity in Semiconductor Gas Sensors", S. R. Morrison, Sensors and Actuators 12(1987) 425-440; and "Electrical Conduction in Solid State Gas Sensors", J. W. Gardner, Sensors and Actuators 18(1989) 373-387.

All such gas sensors rely on the gaseous medium under observation impinging on a surface of a body of the semiconducting metal oxide material and then undergoing some reaction with it which affects the conductance of the semiconducting metal oxide material, which is detected by means of at least one pair of electrodes which are formed upon the body of semiconducting metal oxide material. Hence their performance can be affected by the presence of substances, gaseous or otherwise (other than the materials to be detected), that affect the surface chemistry of the body of semiconducting metal oxide material. It is important to be able to detect when such spurious effects are occuring.

The present invention is based upon the fact that in a perfectly operating sensor, at a given temperature the ratio of the resistance between a first pair of electrodes which form part of the sensor and that between a second pair of electrodes, which also form part of the sensor, but which have a different separation than that between the first pair of electrodes, should vary in a consistent way as a function of the concentration of one gaseous medium, to which the sensor material is adapted to respond, when in the presence of another.

If, however, any changes occur in the surface chemistry of the sensor body (which may or may not be of semiconducting metal oxide material, and which carries the electrodes), such as may be caused by a poisoning material, then the ratio of the two resistances corresponding to various compositions of the gaseous mixture will no longer vary in the same way as before. Hence by making continuous measurements of the ratio of the resistances between the two sets of electrodes and comparing the changes in the ratio of the resistances with a calibration curve, one can distinguish between changes due to real changes in the composition of the gaseous medium under observation and spurious changes due to changes in the performance of the sensor.

According to the invention in a first aspect, therefore, a gas sensor, for determining the presence of a first gaseous medium in a second gaseous medium, is characterised by: a body of an electrically conductive material having an electrical conductivity sensitive to the presence of the first gaseous medium in the second gaseous medium, electrodes disposed in sets on the said body and comprising at least a first set and a second set, the distance between the electrodes of a set of electrodes, or the relationship between the electrodes of a set and an active surface of the said body, being different in the case of each said set, and means for determining the relationship between the resistance between the electrodes in the first set and that between the electrodes in the second set, so that the composition of the gaseous mixture comprising the said media can be determined from that relationship.

Preferably there is an electrode common to more than on set.

The sensor may be constructed in various different ways. It may for example be of a planar or a cylindrical configuration.

In a preferred embodiment of the invention, the pair of electrodes used include a common electrode situated asymmetrically in relation to an electrode of one set and an electrode of another set. This arrangement is for example applicable to both the planar and the cylindrical form of the sensor.

In another arrangement, the electrically conductive body is in the form of a porous disc with a common electrode formed over one planar surface, a central disc electrode on the other planar surface of the disc and an annular electrode surrounding the central disc electrode and concentric with it.

A preferred material for use in the present invention comprises a semiconducting metal oxide ceramic material, which may be in the form of a single such oxide or a mixture of such oxides. Examples of such oxides are tin (IV) oxide, zinc oxide, tungsten (VI) oxide and the oxides described in U.K. patent specifications Nos. GB-A-2 149 120; GB-A-2 149 121; GB-A-2 149 122; GB-A-2 149 123; GB-A-2 166 244. The above oxides can be made to be catalytic for a combustion reaction for use in the performance of the present invention by providing a thin surface coating of particles of one of the well-known catalytic metals such as Pt or Pd; alternatively, they can be made to be catalytic for a decomposition reaction by providing a coating of a suitable material. A decomposition catalyst may be chosen that is specific to a selected gas. Also, the semiconducting metal oxide material can be chosen to be sensitive to a decomposition product of a selected gas.

According to the invention in a second aspect, a method for determining, in a gaseous mixture, the presence of a first gaseous medium in a second gaseous medium, is characterised by the operations of:
(1) Exposing to the second gaseous medium an active surface of the body of a gas sensor according to the invention in its first aspect;
(2) measuring as a function of time the electrical resistances between a pair of electrodes of the first set and between a pair of electrodes of the second set of the sensor; and
(3) using the measured values of the said resistances to compare the relationship between them with a calibration curve indicative of variation of the ratio between the said resistances with concentration of the first gaseous medium in the second gaseous medium, whereby to determine the composition of the gaseous mixture and to detect malfunctioning of the sensor.

Examples of gases the presence of which may be detected by the present invention include hydrocarbons such as methane, ethane, propane, butane, ethylene, benzene and toluene; carbon monoxide; hydrogen; ammonia; hydrogen sulphide; nitrogen dioxide; sulphur dioxide; alcohol vapours such as those of methanol and ethanol; and aldehyde and ketone vapours such as those of formaldehyde, acetone and methyl ethyl ketone.

Embodiments of the invention will now be described, by way of example only and with reference to the accompanying drawings, in which:-
Figure 1 is in two parts denoted (a) and (b), which are a plan view and a cross sectional view, respectively, of a gas sensor in a first embodiment of the invention;
Figure 2 is again in two parts denoted (a) and (b), which are a cross sectional view and a plan view, respectively, of a gas sensor in a second embodiment of the invention;
Figure 3 is a general view of a gas sensor in a third embodiment of the invention;
Figure 4 shows, for the sensor of Figure 1, the variation of the ratio of the resistance measured between widely spaced electrodes to that measured between closely spaced electrodes, as a function of gas concentration for reactive and unreactive gases;
Figure 5 shows, for the sensor of Figure 2, a three-dimensional plot of the ratio of the resistance measured between a common electrode and an inner disc electrode to that measured between the common electrode and an outer ring electrode, as a function of gas concentration and temperature; and
Figure 6 is a plot, in respect of planar sensors as shown in Figure 1, of the variable shown in Figure 4 for a sensor which is working perfectly and one which is not.

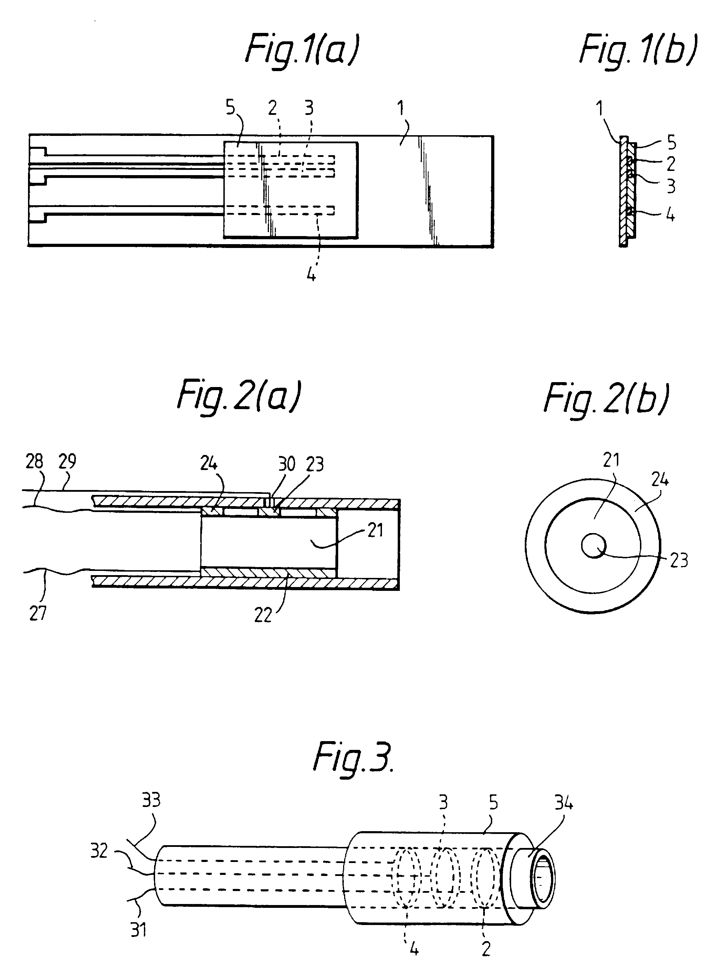

The sensor shown in Figure 1 consists of a gas-impermeable substrate 1 such as a piece of alumina, upon which are deposited three electrodes 2, 3 and 4. These constitute two sets of electrodes 2, 3 and 3, 4, the electrode 3 being common to both sets. The common electrode 3 is asymmetrical with respect to the electrodes 2 and 4, i.e. it is closer to the electrode 2 than to the electrode 4. A body of semiconducting metal oxide material partly covers the electrodes 2, 3 and 4 and constitutes a sensing element 5. The sensing element 5 is porous and has an electrical conductivity which is sensitive to a gas to be detected by the sensor. Its outer surface is active, i.e. exposed to the gaseous environment. If necessary a catalytic layer, not shown, can be deposited on the sensing element 5 to ensure that this gas either burns or is decomposed, so as to cause a change to occur in the conductivity of the sensing element 5.

In the sensor shown in Figure 2, the sensing element is in the form of a disc 21 of porous semiconducting metal oxide material. A metal electrode 22 covers one flat face of the sensing element 21. On the other flat face of the sensing element 21, a central disc electrode 23 and an annular outer electrode 24 are arranged coaxially. The sensing element 21, together with its electrodes 22, 23 and 24, are sandwiched between two flat, parallel, impervious insulating tiles. Contact leads 27 and 28 are attached to the edges of the electrodes 22 and 24 respectively, and a further lead 29 is attached to the electrode 23 via a hole 30 in the corresponding tile. The active surface of the element 21 is here its outer cylindrical surface, which is exposed.

In Figure 2, it can be seen that the relationship between the set of electrodes 22, 23 and the active surface is different from that between the set of electrodes 22, 24 and the same surface, and that the electrode 24 is close to the latter, whereas the electrode 23 is as far away from it as is possible with this configuration.

Figure 3 shows a sensor of tubular geometry, but in other respects it is similar to the sensor of Figure 1. In Figures 1 and 3, corresponding elements have corresponding reference numerals. It should be noted that in practice, the common electrode 3 is closer to one of the electrodes 2, 4 than to the other. Contact with the electrodes 2, 3 and 4 in Figure 3 is made via leads 31, 32 and 33 respectively, which run inside the tubular substrate 34, the outer surfaces of which are active.

In describing the operation of such devices, it is necessary to introduce two parameters Kp and K_{T}. Kp is a measure of the sensitivity of the material of the sensing element to a given gas and hence the concentration of that gas in a gaseous medium under test. K_{T} is a measure of the reactivity and the rate of diffusion, through the sensing element, of the gas or of products of its combustion or decomposition. K_{T} is a function of the operating temperature of the sensor, and this gives the opportunity to use one sensor for the detection of different gases in a mixture by varying the operating temperature of the sensor.

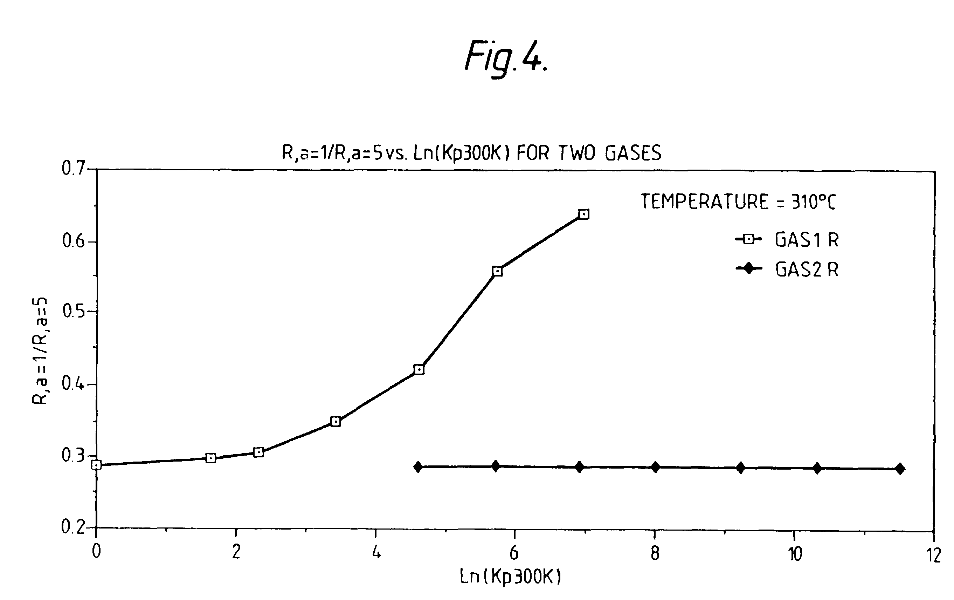

Reference is now made to Figure 4, in which the horizontal co-ordinate axis represents a natural logarithm of Kp, while the vertical axis represents the ratio of the resistance R, a = 1 measured between the electrodes 3 and 4 to the resistance R, a = 5 measured between the electrodes 2 and 3. Figure 4 shows the variation of this ratio as a function (Kp) of the concentration of two gases, for the sensor of Figure 1 at 310°C. Gas 1 is a reactive gas such as carbon monoxide or hydrogen. Gas 2 is an unreactive gas such as methane. Both curves were obtained at the same temperature, so that K_{T} is constant in both cases.

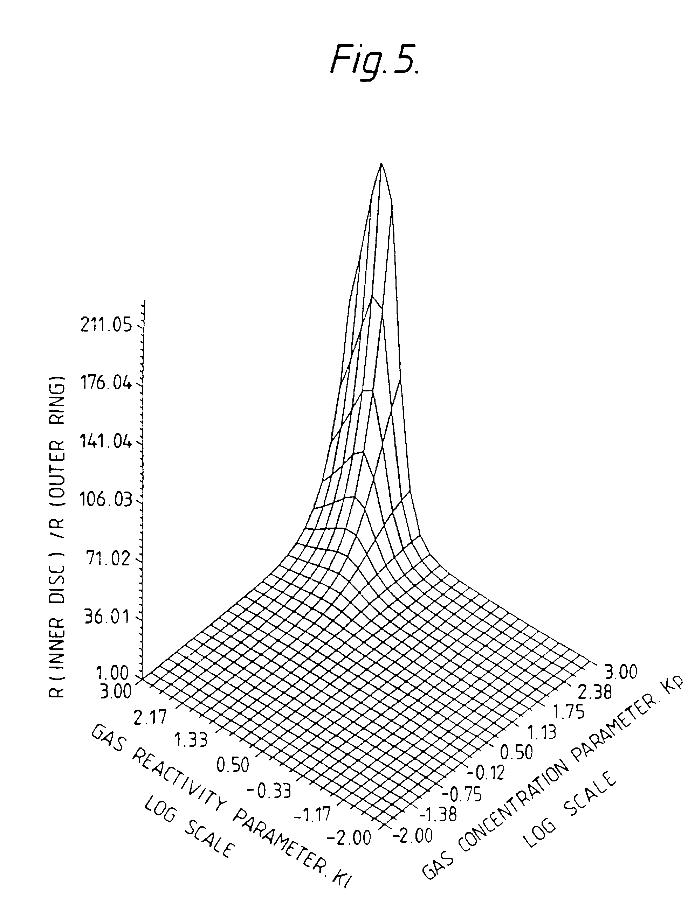

Figure 5, on the other hand, shows for the sensor of Figure 2 a three-dimensional plot of the ratio of the resistance (R [inner disc]) between the inner disc 23 and the common electrode 22 and the resistance (R [outer ring]) between the annular outer electrode 24 and the electrode 22, with variation of both the gas concentration parameter Kp and the gas reactivity or diffusion rate parameter K_{T.}

Both Figures 4 and 5 show that, for unreactive gases (which have a low diffusion parameter K_{T}), the ratio of resistances between the different pairs of electrodes is independent of gas concentration, whereas for reactive gases (which have a high diffusion parameter K_{T}) the ratio of the resistances between the different pairs of electrodes varies considerably with the concentration of the gas. Any zero drift of the sensor is of course cancelled out in the respective resistance ratios.

If a further number of electrodes at different spacings are used in a planar sensor (e.g. as in Figure 1), or in different radial positions in a disc sensor (e.g. as in Figure 2), then taking appropriate ratios will allow the measurement of multiple gases in mixtures to be made. This is because it will usually be possible, especially if use is also made of the possibility of varying the temperature, that one particular gas in the mixture has a composition gradient extending across the gas-sensitive part of the sensor, whereas the other gases in the mixture are either uniform in concentration throughout the gas-sensitive part of the sensor, or have a concentration which falls rapidly to zero at the outer surface of the sensing element.

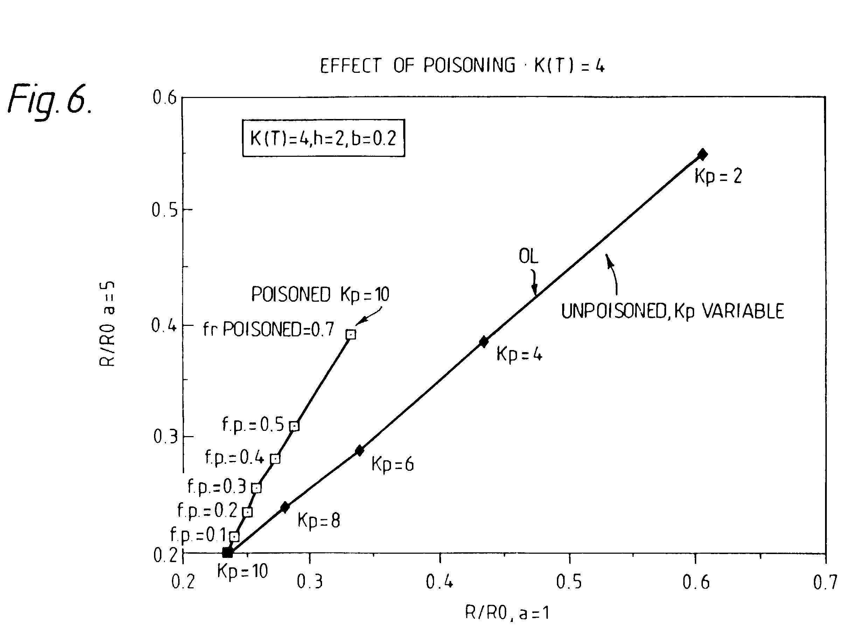

Reference is now made to Figure 6, in which the vertical and horizontal co-ordinate axes represent, respectively, the resistance measured between the more widely-spaced electrodes 3 and 4 and the resistance measured between the closer electrodes 2 and 3, with Kp (the concentration of the reactive gas) as parameter. The value of K_{T} is fixed, as Figure 6 refers to a single gas at a fixed sensor temperature.

Figure 6 relates to a planar sensor such as that shown in Figure 1. If the sensor is operating correctly, then the resistance between the two pairs of electrodes will move along the right-hand line shown as the concentration Kₚ of the reactive gas changes. This line may be referred to as the "operating line". If something other than the concentration of the reactive gas changes, then the measured operating point will move off the expected operating line and the operating line of the sensor as measured will change also.

Also shown in Figure 6 is a measured operating line, where the sensing element of a sensor according to the invention has become poisoned to varying degrees such that, in the outer part of the sensing element extending inwardly from its surface through some fraction of its thickness, the reactive gas does not burn and the conductivity of the material of the sensing element may not respond to the presence of the reactive gas.

Thus, should a point defined by measured resistances be found to be off an operating line obtained under perfect conditions as a calibration curve, then there is indicated a change in conditions other than a change in the concentration of the reactive gas. Factors other than the poisoning of the sensitive element can cause such a change. These include drifts in the zero resistance of the sensor and the presence of reactive gases other than a specific reactive gas. The use of sensors according to the invention and the taking of repeated measurements to derive a measured operating line enables such changes, representing malfunctions of the sensor, to be distinguished from changes in the concentration of the specific reactive gas that is to be detected. This reduces the possibility of false alarms if a sensor is being used to monitor the composition of the given mixture, or can give an indication that a sensor has become faulty and needs to be changed. Thus the measured resistances R can be used to compare the relationship between them with the calibration curve, with a view to establishing the concentration of the gas to be measured, and with the facility to check the result for reliability.

In circumstances where it is known that progressive sensor poisoning takes place, a more elaborate arrangement (not illustrated) enables the progression of the poisoning of the sensor to be followed, and a warning given when it is no longer performing usefully. Instead of two pairs of electrodes being used, there are in this case three pairs of electrodes, with narrow, intermediate and wide spacings. There are now two operating lines, defined by "narrow/intermediate" and "narrow/wide" electrode spacing resistance ratios. Poisoning of the sensitive element will affect the narrow/wide operating line first, and the changes in this operating line will chart the progress of the poisoning. Eventually the narrow/intermediate operating line will begin to be affected. The onset of this change can be used to trigger a suitable warning device.

If three pairs of electrodes are used, then the three resistances define an operating surface instead of an operating line, and a measured operating point would move off this surfce in the event of poisoning of the sensor.

It is evident that the above statements apply equally to the disc configuration of Figure 2, with the inner disc 23 corresponding to the closer electrodes 2 and 3 of the planar version in Figure 1, and with concentric ring electrodes of increasing radius then corresponding to progressively more widely-spaced electrodes of the planar version.

So far as the material of the sensitive layer is concerned, any of the materials listed above can be used. A particular material is chosen in relation to a specific reactive gas to be detected. For example, if it is desired to detect methane in air, then tin dioxide is a suitable material for the sensitive layer. Carbon monoxide in air may be detected also using tin dioxide for the sensitive layer.

## Claims

1. A gas sensor for determining the presence of a first gaseous medium in a second gaseous medium, comprising: a body (5; 21) of an electrically conductive material having an electrical conductivity sensitive to the presence of the first gaseous medium in the second gaseous medium, electrodes disposed in sets on the said body and comprising at least a first set (2, 3; 22, 23) and a second set (3, 4; 22, 24), the distance between the electrodes of a set of electrodes, or the relationship between the electrodes of a set and an active surface of the said body, being different in the case of each said set, and means for determining the relationship between the resistance between the electrodes in the first set and that between the electrodes in the second set, so that the composition of the gaseous mixture comprising the said media can be determined from that relationship.

2. A sensor according to Claim 1, characterised by an electrode (3; 22) common to more than one said set.

3. A sensor according to Claim 2, characterised in that the common electrode (3; 22) is disposed asymmetrically in relation to an electrode (2; 23) of one set and an electrode (4; 24) of another set.

4. A sensor according to any one of the preceding Claims, characterised in that the sensor is of planar configuration, the said body (5) being generally planar with the electrodes (2 to 4) being disposed in contact with a flat surface of the body.

5. A sensor according to any one of Claims 1 to 3, characterised in that the sensor is of cylindrical configuration, the said body (5) being generally cylindrical with the electrodes (2 to 4) being disposed in contact with a cylindrical surface of the body.

6. A sensor according to Claim 2 or Claim 3, characterised in that the electrically conductive body is in the form of a porous disc (21) with an electrode (22), common to each said set, formed over one planar surface, a central disc electrode (23) of one said set on its other planar surface, and an annular electrode (24) of another said set surrounding the central disc electrode and concentric with it.

7. A sensor according to any one of the preceding Claims, characterised in that the body (5; 21) is made of at least one semiconducting metal oxide ceramic material.

8. A sensor according to Claim 7, characterised in that the body (5; 21) has a thin coating of a catalytic material suitable for catalysing at least one type of reaction in the group comprising combustion and decomposition reactions.

9. A method for determining, in a gaseous mixture, the presence of a first gaseous medium in a second gaseous medium, characterised by the operations of:
(1) Exposing to the second gaseous medium an active surface of the body (5; 21) of a gas sensor according to any one of the preceding Claims;
(2) measuring as a function of time the electrical resistances between a pair of electrodes of the first set (2, 3; 22, 23) and between a pair of electrodes of the second set (3, 4; 22, 24) of the sensor; and
(3) using the measured values of the said resistances to compare the relationship between them with a calibration curve indicative of variation of the ratio between the said resistances with concentration of the first gaseous medium in the second gaseous medium, whereby to determine the composition of the gaseous mixture and to detect malfunctioning of the sensor.

## Patentansprüche

1. Gassensor zum Bestimmen des Vorhandenseins eines ersten gasförmigen Mediums in einem zweiten gasförmigen Medium, mit einem Körper (5; 21,) aus einem elektrisch leitenden Material, dessen elektrische Leitfähigkeit auf das Vorhandensein des ersten gasförmigen Mediums in dem zweiten gasförmigen Medium anspricht, mit Elektroden, die satzweise auf dem Körper angeordnet sind und zumindest einen ersten Satz (2, 3; 22, 23) und einen zweiten Satz (3, 4; 22, 24) aufweisen, wobei der Abstand zwischen den Elektroden eines Satzes von Elektroden oder die Relation zwischen den Elektroden eines Satzes und einer aktiven Oberfläche des Körpers für jeden Satz unterschiedlich ist, und mit Mitteln zum Bestimmen der Beziehung zwischen dem Widerstand zwischen den Elektroden im ersten Satz und dem zwischen den Elektroden im zweiten Satz, so daß die Zusammensetzung der gasförmigen Mischung, die die Medien enthält, aus dieser Relation bestimmt werden kann.

2. Sensor nach Anspruch 1, gekennzeichnet durch eine Elektrode (3; 22), die mehr als einem Satz gemeinsam ist.

3. Sensor nach Anspruch 2, dadurch gekennzeichnet, daß die gemeinsame Elektrode (3; 22) gegenüber einer Elektrode (2; 23) das einen Satzes und einer Elektrode (4; 24) des anderen Satzes asymmetrisch angeordnet ist.

4. Sensor nach einem der vorhergehenden Ansprüche, dadurch gekennzeichnet, daß der Sensor von ebener Konfiguration ist, wobei der Körper (5) im wesentlichen eben ist, wobei die Elektroden (2 bis 4) in Kontakt mit einer flachen Fläche des Körpers sind.

5. Sensor nach einem der Ansprüche 1 bis 3, dadurch gekennzeichnet, daß der Sensor von zylindrischer Konfiguration ist, wobei der Körper (5) im wesentlichen zylindrisch ist und die Elektroden (2 bis 4) in Kontakt mit einer zylindrischen Fläche des Körpers sind.

6. Sensor nach Anspruch 2 oder 3, dadurch gekennzeichnet, daß der elektrisch leitende Körper die Form einer porösen Scheibe (21) besitzt, wobei eine Elektrode (22), die jedem Satz gemeinsam ist, längs einer ebenen Fläche gebildet ist, eine mittige Scheibenelektrode (23) eines Satzes auf dessen anderer ebener Fläche ist und eine ringförmige Elektrode (24) eines weiteren Satzes die mittige Scheibenelektrode umgibt und konzentrisch zu ihr ist.

7. Sensor nach einem der vorhergehenden Ansprüche, dadurch gekennzeichnet, daß der Körper (5; 21) aus zumindest einem halbleitenden Metalloxid-Keramik-Material hergestellt ist.

8. Sensor nach Anspruch 7, dadurch gekennzeichnet, daß der Körper (5; 21) einen dünnen Überzug aus einem katalytischen Material besitzt, das zum Katalysieren mindestens einer Reaktionsart in der Gruppe, die Verbrennungs- und Zersetzungsreaktionen enthält, geeignet ist.

9. Verfahren zum Bestimmen das Vorhandenseins eines ersten gasförmigen Mediums in einem zweiten gasförmigen Medium einer gasförmigen Mischung gekennzeichnet durch folgende Schritte:
(1) Eine aktive Oberfläche des Körpers (5; 21) eines Gassensors nach einem der vorhergehenden Ansprüche dem zweiten gasförmigen Medium aussetzen;
(2) Messen der elektrischen Widerstände zwischen einem Paar von Elektroden des ersten Satzes (2, 3; 22, 23) und zwischen einem Paar von Elektroden des zweiten Satzes (3, 4; 22, 24) des Sensors als eine Funktion der Zeit; und
(3) Verwenden der gemessenen Werte dieser Widerstände, zum Vergleichen der Relation zwischen diesen mit einer Eichkurve, die die Änderung des Verhältnisses zwischen den Widerständen mit der Konzentration des ersten gasförmigen Mediums im zweiten gasförmigen Medium anzeigt, wodurch die Zusammensetzung der gasförmigen Mischung bestimmt und eine Fehlfunktion des Sensors erfaßt wird.

## Revendications

1. Détecteur de gaz pour déterminer la présence d'un premier milieu gazeux dans un second milieu gazeux, comprenant : un corps (5 ; 21) fait d'une matière conductrice de l'électricité, ayant une conductivité électrique sensible à la présence du premier milieu gazeux dans le second milieu gazeux, des électrodes disposées en ensembles sur ledit corps et comprenant au moins un premier ensemble (2, 3 ; 22, 23) et un second ensemble (3, 4 ; 22, 24), la distance entre les électrodes d'un ensemble d'électrodes, ou la relation entre les électrodes d'un ensemble et une surface active dudit corps, étant différente dans le cas de chacun desdits ensembles, et des moyens pour déterminer la relation entre la résistance entre les électrodes dans le premier ensemble et celle entre les électrodes dans le second ensemble, de telle sorte que la composition du mélange gazeux comprenant lesdits milieux puisse être déterminée à partir de cette relation.

2. Détecteur selon la revendication 1, caractérisé par une électrode (3 ; 22) commune à plus d'un desdits ensembles.

3. Détecteur selon la revendication 2, caractérisé par le fait que l'électrode commune (3 ; 22) est disposée de façon asymétrique par rapport à une électrode (2 ; 23) d'un ensemble et une électrode (4 ; 24) d'un autre ensemble.

4. Détecteur selon l'une quelconque des revendications précédentes, caractérisé par le fait que le détecteur est de configuration plane, ledit corps (5) étant généralement plan, les électrodes (2 à 4) étant disposées en contact avec une surface plate du corps.

5. Détecteur selon l'une quelconque des revendications 1 à 3, caractérisé par le fait que le détecteur est de configuration cylindrique, ledit corps (5) étant généralement cylindrique, les électrodes (2 à 4) étant disposées en contact avec une surface cylindrique du corps.

6. Détecteur selon la revendication 2 ou la revendication 3, caractérisé par le fait que le corps conducteur de l'électricité se présente sous la forme d'un disque poreux (21) avec une électrode (22), commune à chacun desdits ensembles, formée par-dessus une surface plane, une électrode centrale en forme de disque (23) de l'un desdits ensembles sur son autre surface plane, et une électrode annulaire (24) d'un autre desdits ensembles entourant l'électrode centrale en forme de disque et étant concentrique avec elle.

7. Détecteur selon l'une quelconque des revendications précédentes, caractérisé par le fait que le corps (5 ; 21) est fait d'au moins une matière céramique de type oxyde métallique semi-conducteur.

8. Détecteur selon la revendication 7, caractérisé par le fait que le corps (5 ; 21) a un revêtement mince d'une matière catalytique appropriée pour catalyser au moins un type de réaction dans le groupe comprenant les réactions de combustion et de décomposition.

9. Procédé de détermination, dans un milieu gazeux, de la présence d'un premier milieu gazeux dans un second milieu gazeux, caractérisé par les opérations consistant à :
(1) exposer au second milieu gazeux une surface active du corps (5 ; 21) d'un détecteur de gaz tel que défini à l'une quelconque des revendications précédentes ;
(2) mesurer en fonction du temps les resistances électriques entre une paire d'électrodes du premier ensemble (2, 3 ; 22, 23) et entre une paire d'électrodes du second ensemble (3, 14 ; 22, 24) du détecteur ; et
(3) utiliser les valeurs mesurées desdits résistances pour comparer la relation entre elles avec une courbe d'étalonnage indicatrice de la variation du rapport entre lesdits résistances avec la concentration du premier milieu gazeux dans le second milieu gazeux, permettant ainsi de déterminer la composition du milieu gazeux et de détecter un fonctionnement défectueux du détecteur.
